# EUROPÄISCHE PATENTSCHRIFT

(11) **EP 1 068 062 B1**
(45) Veröffentlichungstag und Bekanntmachung des Hinweises auf die Patenterteilung: **12.06.2002**
(21) Anmeldenummer: 99917917.9
(22) Anmeldetag: 30.03.1999
(51) Int. Cl.: B29C 63/02, B29C 63/48, G01N 33/44

(54) **VERFAHREN UND VORRICHTUNG ZUM BESCHICHTEN EINER OBERFLÄCHE MIT EINER KUNSTSTOFFOLIE**
METHOD AND DEVICE FOR COATING A SURFACE WITH A PLASTIC FILM
PROCEDE ET DISPOSITIF DE REVETEMENT D'UNE SURFACE PAR UNE FEUILLE DE MATIERE PLASTIQUE

(30) Priorität: 31.03.1998 DE 19814390
(43) Veröffentlichungstag der Anmeldung: 17.01.2001
(73) Patentinhaber: Advanced Photonics Technologies AG, 83052 Bruckmühl-Heufeld (DE)
(72) Erfinder: BÄR, Kai, K., O., D-83052 Bruckmühl-Heufeld (DE); GAUS, Rainer, D-83052 Bruckmühl-Heufeld (DE)
(74) Vertreter: Bohnenberger, Johannes, Dr.
(86) Internationale Anmeldenummer: EP9902173
(87) Internationale Veröffentlichungsnummer: WO9950048

(56) Entgegenhaltungen:
- EP-A- 0 369 998
- PATENT ABSTRACTS OF JAPAN vol. 002, no. 144 (C-029), 30. November 1978 (1978-11-30) & JP 53 108172 A (SEKISUI CHEM CO LTD), 20. September 1978 (1978-09-20)
- PATENT ABSTRACTS OF JAPAN vol. 007, no. 201 (C-184), 6. September 1983 (1983-09-06) & JP 58 101177 A (MODAN PLASTIC KOGYO KK), 16. Juni 1983 (1983-06-16)
- PATENT ABSTRACTS OF JAPAN vol. 096, no. 001, 31. Januar 1996 (1996-01-31) & JP 07 241910 A (NISSAN MOTOR CO LTD), 19. September 1995 (1995-09-19)

## Beschreibung

Die Erfindung betrifft das Beschichten einer Oberfläche mit einer Kunststoffolie, insbesondere der Oberfläche einer PKW-Stoßstange. Insbesondere wird beim Beschichten der Oberfläche die Oberfläche und/oder die Kunststoffolie befeuchtet, so daß die Oberfläche nach dem Aufbringen der Kunststoffolie zunächst zumindest stellenweise durch einen Feuchtigkeitsfilm von der Kunststoffolie getrennt ist, und wobei der Feuchtigkeitsfilm zumindest teilweise durch Ausrakeln entfernt wird. Die Erfindung betrifft weiterhin die Qualitätssicherung bei der Ausführung eines Verfahrens zum Beschichten einer Oberfläche mit einer Kunststoffolie, insbesondere der Oberfläche einer PKW-Stoßstange.

Aus unterschiedlichen Gründen werden heutzutage vielfach die Oberflächen von Gegenständen mit Kunststoffolien beschichtet. Beispielsweise werden durch die Kunststoffolien empfindliche Oberflächen gegen Verkratzen und gegen Chemikalieneinwirkung geschützt, Farbwirkungen durch Interferenzeffekte erzielt oder der Glanzgrad erhöht. In der Automobilindustrie ist es beispielsweise üblich, Stoßbänder bzw. Stoßstangen an Personenkraftwagen (PKW) anzubringen, die farblich auf die Lackierung des PKW abgestimmt sind. Die Stoßstangen bestehen aus einem Kunststoffmaterial, das die gewünschte Farbe hat, jedoch häufig aus verhältnismäßig weichem und flexiblem Material besteht, so daß die Oberfläche unter den hohen Beanspruchungen des Alltags leicht verkratzt. Hinzu kommt, daß die Kratzer auf glatten, vorzugsweise glänzenden lackartigen Kunststoffoberflächen wesentlich besser sichtbar sind als auf matten, grobstrukturierten Oberflächen.

Zum Beschichten einer Oberfläche mit einer Kunststoffolie, insbesondere der Oberfläche einer PKW-Stoßstange, ist es aus der JP 58101177 bekannt, die Oberfläche und/oder die Kunststoffolie zu befeuchten, so daß die Oberfläche nach dem Aufbringen der Kunststoffolie zunächst zumindest stellenweise durch einen Feuchtigkeitsfilm von der Kunststoffolie getrennt ist. Die Flüssigkeit, die den Feuchtigkeitsfilm bildet, kann insbesondere eine Spülmittellösung sein. Der Feuchtigkeitsfilm erlaubt es, die Kunststoffolie genau in der gewünschten Weise auf der Oberfläche zu positionieren bzw. zurechtzurücken. Nachdem die Kunststoffolie an der richtigen Stelle bzw. in dem richtigen Bereich an der Oberfläche positioniert ist, wird der Feuchtigkeitsfilm zumindest teilweise, vorzugsweise annähernd vollständig, durch Ausrakeln entfernt.

Ein Teil der Feuchtigkeit wird meistens an der Oberfläche und/oder an der Kunststoffolie gebunden. Ein weiterer Teil kann mit einem Klebstoff, der ebenfalls zwischen der Kunststoffolie und der Oberfläche angeordnet ist, eine Verbindung eingehen. In vielen Fällen dient die Feuchtigkeit sogar dazu, den Klebstoff zu aktivieren, so daß eine wirksame und dauerhafte Klebewirkung erzielt wird.

Wird beim Ausrakeln jedoch der überschüssige Teil der Feuchtigkeit nicht weitgehend entfernt, kann es zu deutlich sichtbaren Unregelmäßigkeiten der Beschichtung kommen. Insbesondere können sich über einen Zeitraum von mehreren Stunden hinweg Feuchtigkeitsgehalte, die in einer der beschriebenen Weisen gebunden waren oder die verhältnismäßig gleichmäßig über die Oberfläche verteilt waren, sammeln und zu Blasenbildung führen. Dies stellt - insbesondere bei der heutzutage üblichen Just-in-time-Anlieferung von Zubehörteilen - für die Automobilfertigung ein großes Problem dar. Unter Umständen müssen in verhältnismäßig kurzer Zeit große Stückzahlen von beispielsweise einer PKW-Stoßstange gefertigt und angeliefert werden, wobei etwaige Fehler beim Beschichten erst nach Anlieferung oder gar erst nach Montage der Stoßstange zutage treten.

Ein weiteres Problem stellen Unregelmäßigkeiten der Kunststoffolie und/oder der bereits vor dem Beschichten an der Kunststoffolie oder der Oberfläche angeordneten Klebstoffschicht dar. Insbesondere kann die Schichtdicke oder Materialstruktur der Kunststoffolie ungleichmäßig sein. Derartige Unregelmäßigkeiten der Kunststoffolie und/oder einer Klebstoffschicht werden häufig erst sichtbar, wenn die Kunststoffolie bereits auf die Oberfläche des zu beschichtenden Gegenstandes aufgebracht worden ist. Dies liegt insbesondere daran, daß die Kunststoffolie in vielen Fällen durchsichtig ist und erst anhand von Unregelmäßigkeiten des von der beschichteten Oberfläche reflektierten oder gebrochenen Lichts der Fehler festgestellt werden kann. Weiterhin kann insbesondere durch das Ausrakeln des Feuchtigkeitsfilms zwischen der Kunststoffolie und der Oberfläche eine ungleichmäßige Verteilung des Klebstoffes erzeugt werden.

Aus der EP 0 369 998 ist eine Vorrichtung zum Beschichten einer Oberfläche, nämlich der Innenwandung eines fest verlegten Rohres, mit einer Kunststofffolie bekannt, welche eine Strahlungsquelle zum Erzeugen von Infrarotstrahlung zum Erwärmen der Beschichtung und einen Reflektor zum Erhöhen der Strahlungsflußdichte der von der Strahlungsquelle ausgehenden Infrarotstrahlung aufweist. Als Strahlungsquelle wird ein im Bereich des mittleren Infrarot arbeitender IR-Heizer eingesetzt.

Aufgabe der vorliegenden Erfindung ist es, ein Verfahren zum Beschichten einer Oberfläche mit einer Kunststoffolie, insbesondere der Oberfläche einer PKW-Stoßstange der eingangs genannten Art anzugeben, bei dem eine möglichst geringe Zahl von fehlerhaften Produkten ausgeliefert wird. Dabei sollen etwaige Fehler möglichst rasch erkannt werden und nach Möglichkeit geringfügige Unregelmäßigkeit der Beschichtung beseitigt werden. Eine weitere Aufgabe der vorliegenden Erfindung ist es, eine Vorrichtung zum Beschichten einer Oberfläche mit einer Kunststoffolie anzugeben, mit der die genannten Anforderungen erfüllbar sind. Außerdem ist es Aufgabe der vorliegenden Erfindung, einen Gegenstand anzugeben, durch dessen Verwendung die genannten Anforderungen erfüllbar sind.

Die Aufgaben werden durch ein Verfahren mit den Merkmalen des Anspruchs 1, durch eine Vorrichtung mit den Merkmalen des Anspruchs 7 und durch eine Verwendung mit den Merkmalen des Anspruchs 12 gelöst. Weiterbildungen sind Gegenstand der jeweils abhängigen Ansprüche.

Ein wesentlicher Gedanke bei der hier vorliegenden Erfindung besteht darin, daß die Beschichtung der Oberfläche während und/oder nach dem Ausrakeln des Feuchtigkeitsfilms mit Infrarotstrahlung bestrahlt wird, um zumindest Teile der Beschichtung und/oder gegebenenfalls verbliebene Restfeuchtigkeit zwischen der Oberfläche und der Kunststoffolie zu erwärmen.

Ist eine solche Restfeuchtigkeit vorhanden, werden durch die Erwärmung Prozesse beschleunigt, die ohne Erwärmung über einen langsamen Zeitraum hinweg ablaufen würden oder erst zu einem späteren, nicht voraussagbaren Zeitpunkt ausgelöst werden, wenn beispielsweise die Beschichtung extremen Umwelteinflüssen wie hohe Umgebungstemperatur oder intensive Sonneneinstrahlung ausgesetzt wird. Es kann daher in kurzer Zeit festgestellt werden, ob die Beschichtung vorgegebenen Qualitätsansprüchen genügt.

Die Infrarotstrahlung kann von der Kunststoffolie selbst und/oder der Restfeuchtigkeit und/oder einer etwaig vorhandenen Klebstoffschicht absorbiert werden. Vorzugsweise jedoch wird der größte Anteil der eingestrahlten Energie unmittelbar im Bereich zwischen der Oberfläche des zu beschichtenden Gegenstandes und der Kunststoffolie absorbiert. Insbesondere bei kurzzeitiger Bestrahlung mit hohen Strahlungsflußdichten wird dann die Erwärmung an der gewünschten Stelle bewirkt, ohne daß die Kunststoffolie und der zu beschichtenden Gegenstand nennenswert erwärmt werden. Meist handelt es sich bei den Kunststoffolien um temperaturempfindliche Folien, die oberhalb einer Schädigungstemperatur thermoplastisch verformt werden. Bei der genannten bevorzugten Ausführungsform kann also mit großer Sicherheit eine Schädigung der Kunststoffolie und/oder des zu beschichtenden Gegenstandes vermieden werden.

Besonders bevorzugt wird eine Weiterbildung, bei der ein weitgehend kontinuierliches Strahlungspektrum eingestrahlt wird, dessen spektrales Strahlungsdichtemaximum in einem Wellenlängenbereich jenseits des sichtbaren Wellenlängenbereichs bis zu einer Wellenlänge von 1,4 µm liegt. Dieser Bereich wird als nahes Infrarot bezeichnet.

Bevorzugt wird eine Weiterbildung des Verfahrens, bei der die gegebenenfalls verbliebene Restfeuchtigkeit durch die Infrarotstrahlung unter Blasenbildung gesammelt wird. Somit können bereits während oder bereits kurze Zeit nach der Bestrahlung Fehler beim Aufbringen der Kunststoffolie bzw. beim Ausrakeln des Feuchtigkeitsfilms erkannt werden. Die Produkte können ausgesondert und/oder neu beschichtet werden. Gegenüber dem in der Beschreibungseinleitung beschriebenen bekannten Verfahren kann die Qualitätskontrolle unmittelbar nach der eigentlichen Beschichtung stattfinden. Eine spätere Qualitätskontrolle, etwa in einer PKW-Produktionsstraße, kann daher entfallen, unter Einsparung von Arbeitszeit und Personalkosten.

Häufig wird eine zwischen der Oberfläche und der Kunststofffolie angeordnete Klebstoffschicht zur Befestigung der Kunststoffolie verwendet. Vorzugsweise wird in solchen Fällen die Beschichtung derart bestrahlt, daß die Klebstoffschicht durch die Bestrahlung erwärmt wird und gleichmäßig verläuft. Es entsteht eine überall gut haftende Beschichtung, bei der die Klebstoffschicht zu keinerlei optisch erkennbaren Unregelmäßigkeit führt.

Ist mit Ungleichmäßigkeiten der Kunststoffolie, wie ungleichmäßige Schichtdicke oder ungleichmäßige Materialstruktur, zu rechnen, werden durch die Bestrahlung insbesondere geringfügige Ungleichmäßigkeiten ausgeglichen oder zumindest annähernd ausgeglichen. Hierbei findet insbesondere auch in der Kunststoffolie eine nennenswerte Absorption der Infrarotstrahlung statt, so daß eine Homogenisierung, beispielsweise eine gleichmäßige Polymerisierung, stattfindet. Die Unregelmäßigkeiten können aber auch lediglich an der Oberfläche der Kunststoffolie auftreten, so daß vorzugsweise in diesem Bereich der wesentliche Teil der Strahlungsenergie absorbiert wird und zu einer Glättung der Folienoberfläche führt.

Bei der Beschichtung einer PKW-Stoßstange mit einer sich etwa über die gesamte Breite und Länge der Stoßstange erstreckenden Kunststoffolie wird die Beschichtung vorzugsweise insgesamt weniger als 20 s lang, insbesondere weniger als 10 s lang bestrahlt, wobei bevorzugtermaßen Infrarotstrahlung aus einer Halogenlampe mit einer Glühkörper-Oberflächentemperatur von mehr als 2500 K verwendet wird.

Die erfindungsgemäße Vorrichtung zum Beschichten einer Oberfläche mit einer Kunststoffolie, insbesondere der Oberfläche einer PKW-Stoßstange, weist eine Strahlungsquelle zum Erzeugen von Infrarotstrahlung und einen Reflektor zum Erhöhen der Strahlungsflußdichte der von der Strahlungsquelle auf die Beschichtung eingestrahlten Infrarotstrahlung auf. Die Strahlungsquelle weist zumindest eine Halegenlampe auf, die insbesondere bei Oberflächentemperaturen von mehr als 2500 K betreibar ist. Mit der Infrarotstrahlung werden zumindest Teile der Beschichtung der Oberfläche und/oder beim Beschichten verwendete Hilfsstoffe, wie Flüssigkeiten oder Flüssigkeitszusätze erwärmt. Insbesondere läßt sich das oben bereits beschriebene Verfahren in wenigstens einer seiner Varianten ausführen.

Insbesondere bei einer Fertigungsstation zum Beschichten der Oberfläche einer PKW-Stoßstange weist die Vorrichtung vorzugsweise eine Halteeinrichtung zum Halten des zu beschichtenden Gegenstandes und eine Verschiebeeinrichtung zum Verschieben eines auf einen Teilbereich der Beschichtung gerichteten Strahlungsbündels aus der Strahlungsquelle auf. Mit Hilfe der Verschiebeeinrichtung kann das Strahlungsbündel bzw. die Strahlungsquelle entlang der Oberfläche des zu beschichtenden Gegenstandes verschoben werden, um auch andere Teilbereiche der Beschichtung zu bestrahlen. Die Verschiebeeinrichtung wird zweckmäßigerweise durch einen Motor angetrieben, der insbesondere gemäß vorher festgelegter Steuerparameter betrieben wird, so daß eine definierte Steuerung der Verschiebebewegung erfolgt.

Bei einer Weiterbildung der Vorrichtung weist die Verschiebeeinrichtung ein Führungselement, insbesondere eine Schiene auf, entlang dem die Strahlungsquelle verschiebbar ist. Vorzugsweise ist das Führungselement derart entsprechend der Formgebung des zu beschichtenden Gegenstandes gebogen, daß die Strahlungsquelle bei gleichbleibendem Abstand zu der zu beschichtenden Oberfläche des Gegenstandes verschiebbar ist. In diesem Fall ist zweckmäßigerweise die Steuerung derart ausgeführt, daß die Strahlungsquelle mit konstanter Geschwindigkeit entlang dem Führungselement verschoben wird.

Ein Vorteil der erfindungsgemäßen Vorrichtung ist, daß der zu beschichtende Gegenstand nicht wie auf einer Fertigungsstraße bewegt wird, sondern festgehalten wird, so daß in Ruhe eine genaue Positionierung der Kunststoffolie an der zu beschichtenden Oberfläche stattfinden kann. Zu gegebener Zeit kann dann die Verschiebeeinrichtung betätigt werden, während der Gegenstand noch von der Halteeinrichtung gehalten wird.

Die Strahlungsquelle weist zumindest eine Halogenlampe auf, die insbesondere bei Oberflächentemperaturen von mehr als 2500 K betreibbar ist. Zur Erzeugung spezieller Strahlungsspektren werden Strahlungsfilter, beispielsweise eine Glasscheibe mit den gewünschten optischen Eigenschaften, zwischen der Strahlungsquelle und der zu bestrahlenden Beschichtung angeordnet. Die Befestigungsstellen des Glühkörpers der Halogenlampe sind vorzugsweise durch Luftstromkühlung zwangsgekühlt. Auf diese Weise kann die Lebensdauer der Halogenlampe deutlich gesteigert werden. Vorzugsweise wird weiterhin der Reflektor durch Flüssigkeitskühlung auf bzw. unterhalb einer vorgegebenen Temperatur gehalten, um die Reflektionseigenschaften des Reflektors konstant zu halten und den Reflektor möglichst keiner thermischen Belastung auszusetzen.

In besonders bevorzugter Ausgestaltung weist die Vorrichtung eine Regeleinrichtung zum Regeln der Beschichtungstemperatur auf, wobei ein auf die Beschichtung ausrichtbares Pyrometer zur Messung der Beschichtungstemperatur vorgesehen ist. Die Regeleinrichtung verhindert insbesondere, daß eine die Kunststoffolie schädigende Wärmeentwicklung auftritt.

Ein weiterer erfindungsgemäßer Gedanke sieht vor, bei der Ausführung eines Verfahrens zum Beschichten einer Oberfläche mit einer Kunststoffolie, insbesondere nach einer Ausgestaltung des zuvor beschriebenen Verfahrens, eine Infrarotlampe zur Qualitätssicherung zu verwenden, wobei die Beschichtung mit Infrarotstrahlung aus der Infrarotlampe bestrahlt wird, um Unregelmäßigkeiten der Beschichtung sichtbar zu machen und/oder zumindest annähernd zu beseitigen. Vorzugsweise ist die Infrarotlampe eine Halogenlampe. In besonderer Ausgestaltung ist die Infrarotlampe als Röhrenstrahler mit einem sich linienartig in einer strahlungsdurchlässigen Röhre, insbesondere in einer Quarzglasröhre, erstreckenden Glühfaden ausgebildet. Zweckmäßigerweise ist die Infrarotlampe mit einem Reflektor kombiniert, der sich längs der Röhre erstreckt und diese im Querschnitt derart rinnenartig an der Rückseite umgibt, daß die in Richtung der Vorderseite abgestrahlte Strahlung durch reflektierte Strahlung verstärkt wird. Somit wird eine unerwünschte Abstrahlung in Richtung der Rückseite vermieden und wird der Wirkungsgrad der Anordnung erhöht. Außerdem kann durch den Reflektor eine spezielle Strahlungsflußdichteverteilung über den bestrahlten Bereich der Beschichtung eingestellt werden, insbesondere eine konstante Strahlungsflußdichteverteilung.

Ausführungsbeispiele der vorliegenden Erfindung werden nun anhand der Zeichnung beschrieben. Die Erfindung ist jedoch nicht auf diese Ausführungsbeispiele beschränkt. Die einzelnen Figuren der Zeichnung zeigen:
- Fig. 1: ein Ausführungsbeispiel für die erfindungsgemäße Vorrichtung,
- Fig. 2: beispielhaft den Aufbau einer Beschichtung vor dem Ausrakeln eines Feuchtigkeitsfilms zwischen der Kunststoffolie und der zu beschichtenden Oberfläche,
- Fig. 3: den Aufbau der Beschichtung nach Fig. 2 nach dem Ausrakeln und
- Fig. 4: den Aufbau der Beschichtung gemäß Fig. 3 unmittelbar nach einer Infrarotbestrahlung.

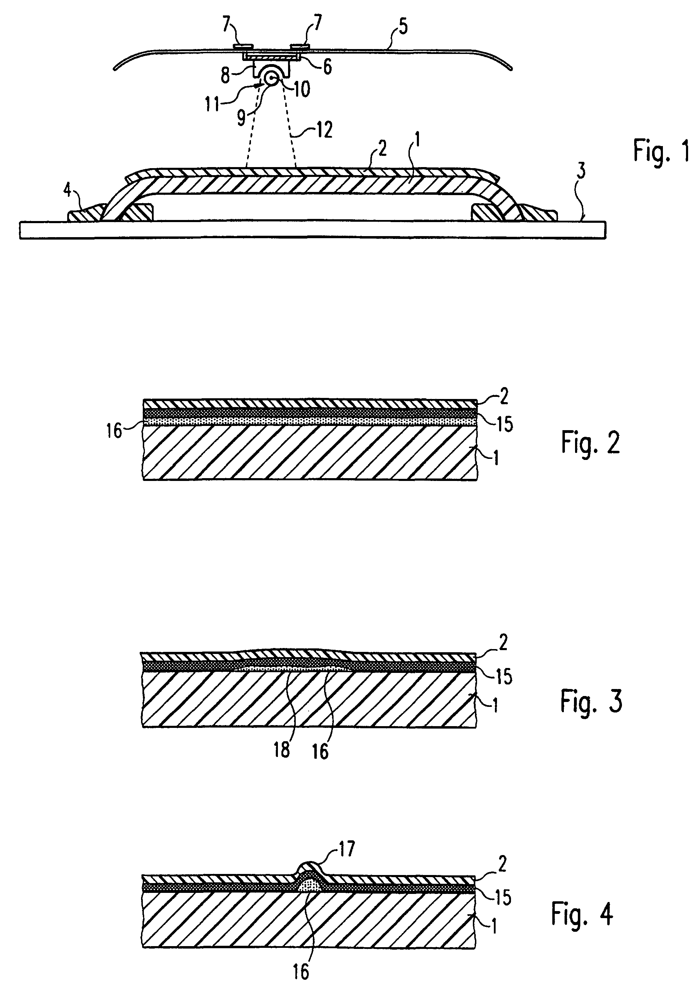

Fig. 1 zeigt eine Ausführungsform der erfindungsgemäßen Vorrichtung mit einer Auflagefläche 3, auf der eine PKW-Stoßstange 1 aufgelegt ist. Die PKW-Stoßstange 1 wird von Haltebacken 4 gehalten, die fest an der Auflagefläche 3 positioniert sind. Bei der PKW-Stoßstange 1 handelt es sich um einen Teil einer Stoßfängereinrichtung, die am Heck eines PKW im Bereich der Laderaumklappe angebracht wird. Die PKW-Stoßstange 1 ist aus farblich auf die Lackierung des PKW abgestimmtem Kunststoffmaterial gefertigt. Insbesondere um ein Verkratzen der PKW-Stoßstange 1 zu verhindern, wird mit der in Fig. 1 gezeigten Vorrichtung eine Polyethylen-(PE-)Folie an der Oberseite der PKW-Stoßstange 1 angebracht. In der Darstellung von Fig. 1 ist die PE-Folie 2 übertrieben dick dargestellt.

Die gezeigte Vorrichtung weist eine Schiene 5 auf, entlang der eine Halogenlampe 11 verschiebbar ist. Die Halogenlampe 11 ist, wie aus der gezeigten Querschnittsdarstellung ersichtlich ist, derart rinnenartig an ihrer Rückseite von einem Reflektor 8 umgeben, daß die in Richtung der Vorderseite abgestrahlte Strahlung durch reflektierte Strahlung verstärkt wird. Der Reflektor 8 ist an einem Lampenhalter 6 befestigt, der wiederum mit zwei Reitern 7 verbunden ist, die durch die Schiene 5 geführt sind und die Verschiebung der Halogenlampe 11 erlauben.

Senkrecht zur Bildebene der Fig. 1 erstrecken sich die Quarzglasröhre und der Glühfaden 10 der Halogenlampe 11 über eine Länge, die etwa der Breite der PE-Folie 2 entspricht. Aufgrund der Kombination der Halogenlampe 11 mit dem Reflektor 8 trifft ein leicht seitlich divergierendes Strahlungsbündel 12 auf einen Teilbereich der Beschichtung der PKW-Stoßstange 1.

Durch Verschieben der Anordnung entlang der Schiene 5 kann die gesamte Beschichtung gleichmäßig überstrichen werden. Die Enden der Schiene 5 sind entsprechend der Formgebung der PKW-Stoßstange 1 so gebogen, daß auch die Endbereiche der PE-Folie 2 aus derselben Entfernung bestrahlt werden können wie der mittlere Abschnitt der PE-Folie 2.

Die Halogenlampe 11 wird vorzugsweise so betrieben, daß die Oberflächentemperatur des Glühfadens 10 etwa 3000 K beträgt. Das Maximum der spektralen Strahlungsflußdichteverteilung liegt daher ungefähr bei 1 µm Wellenlänge, d. h. im nahen Infrarot.

Anhand der Fig. 2-4 wird nun ein Ausführungsbeispiel des erfindungsgemäßen Verfahrens beschrieben, bei der insbesondere die Vorrichtung gemäß Fig. 1 verwendet werden kann. Die Figuren zeigen Querschnitte durch einen bestimmten Teilabschnitt der PKW-Stoßstange 1 gemäß Fig. 1 in verschiedenen Verfahrensstadien.

Zunächst wird die zu beschichtende Oberfläche der PKW-Stoßstange 1 mit einer wäßrigen Spülmittellösung 16 benetzt. Anschließend wird die bereits an ihrer Unterseite mit einer Klebstoffschicht 15 versehene PE-Folie 2 auf die benetzte Oberfläche aufgebracht. Damit ist der in Fig. 2 gezeigte Zustand erreicht. Im Unterschied zu der gezeigten Darstellung kann es vorkommen oder zur Erreichung einer ausreichenden Haftwirkung sogar erforderlich sein, daß die Klebstoffschicht 15 Feuchtigkeit, d. h. Spülmittellösung aufnimmt, um die PE-Folie auf einfache Weise an exakt die gewünschte Position verschieben zu können. Es ist jedoch vorteilhaft, wenn überschüssige Spülmittellösung 16 vorhanden ist, da diese das Eintreten der Haftwirkung des Klebstoffes verhindert oder verzögert und einen Gleitfilm mit niedrigem Reibungskoeffizienten darstellt. Nach dem Aufbringen der PE-Folie 2 wird die Spülmittellösung zumindest weitgehend per Hand oder mit einem geeigneten Werkzeug ausgestrichen bzw. ausgerakelt. Dadurch entfaltet der Klebstoff 15 seine Haftwirkung und fixiert die PE-Folie 2 an der Oberfläche der PKW-Stoßstange 1. Damit ist der in Fig. 3 gezeigte Zustand erreicht, wobei jedoch nach dem Ausrakeln in einem Teilbereich an der Oberfläche der PKW-Stoßstange 1 überschüssige Spülmittellösung 16 als Restfeuchtigkeit 18 verblieben ist. Die Restfeuchtigkeit 18 weist eine geringe Schichtdicke auf, da sie an der Oberfläche der PKW-Stoßstange und/oder an dem Klebstoff 15 bzw. der Unterseite der PE-Folie 2 gebunden ist. Außerdem bildet der Klebstoff 15 insbesondere im Bereich der Restfeuchtigkeit 18 eine ungleichmäßig dicke Schicht.

Würde nun, ohne die Ausführung weiterer Verfahrensschritte, einige Stunden abgewartet, so würde der Klebstoff 15 vollständig abbinden, unter Umständen weitere, gebundene Teilchen der Spülmittellösung freigeben und es würde allmählich eine Ansammlung der Restfeuchtigkeit 18 auf engem Raum entstehen, die als Blase 17, wie in Fig. 4 gezeigt, deutlich von außen sichtbar würde. Eine solche Beschichtung erfüllt nicht die üblichen Qualitätsanforderungen, so daß die PKW-Stoßstange 1 vor der Montage ausgesondert werden muß, bzw. nach der Montage an einem PKW demontiert und ersetzt bzw. neu beschichtet werden müßte.

Erfindungsgemäß wird daher zur Qualitätssicherung die Beschichtung der PKW-Stoßstange 1 während und/oder dem Ausrakeln mit Infrarotstrahlung bestrahlt, so daß zum einen der Klebstoff 15 in gleichmäßiger Dicke über die Oberfläche der PKW-Stoßstange 1 verteilt wird (mit Ausnahme des Bereichs der verbliebenen Restfeuchtigkeit 18) und andererseits die verbliebene Restfeuchtigkeit 18 unter Bildung einer Blase 17 gesammelt wird. Unter "Blasen" werden hier jegliche deutlich sichtbare Erhebungen der Kunststoffolie bezeichnet, die Ursache einer Flüssigkeitsansammlung sind, insbesondere können sich auch längliche, wellenartige Blasen bilden. Somit ist bereits während oder kurz nach der Bestrahlung erkennbar, ob die Beschichtung die Qualitätsanforderungen erfüllt. Weiterhin ist, falls keine überschüssige Restfeuchtigkeit sichtbar gemacht wurde, überall eine gleichmäßig dicke Klebstoffschicht vorhanden. Außerdem können etwaige geringfügige Ungleichmäßigkeiten der Kunststoffolie, die beispielsweise als Schlieren oder wolkenartige Strukturen erkennbar sind, durch die Erwärmung ausgeglichen werden.

### Bezugszeichenliste

- 1: PKW-Stoßstange
- 2: PE-Folie
- 3: Auflagefläche
- 4: Haltebacken
- 5: Schiene
- 6: Lampenhalter
- 7: Reiter
- 8: Reflektor
- 9: Quarzglasröhre
- 10: Glühfaden
- 11: Halogenlampe
- 12: Strahlungsbündel
- 15: Klebstoff
- 16: Spülmittellösung
- 17: Blase
- 18: Restfeuchtigkeit

## Patentansprüche

1. Verfahren zum Beschichten einer Oberfläche mit einer Kunststoffolie (2), insbesondere der Oberfläche einer PKW-Stoßstange (1), wobei die Oberfläche und/oder die Kunststoffolie (2) befeuchtet wird, so daß die Oberfläche nach dem Aufbringen der Kunststoffolie (2) zunächst zumindest stellenweise durch einen Feuchtigkeitsfilm (16) von der Kunststoffolie (2) getrennt ist, und wobei der Feuchtigkeitsfilm (16) zumindest teilweise durch Ausrakeln entfernt wird,
**dadurch gekennzeichnet,**
**daß** die. Beschichtung (2, 15, 16) während und/oder nach dem Ausrakeln mit Infrarotstrahlung (12) bestrahlt wird, um zumindest Teile der Beschichtung (2, 15, 16) und/oder eine gegebenenfalls verbliebene Restfeuchtigkeit (18) zwischen der Oberfläche und der Kunststoffolie (2) zu erwärmen.

2. Verfahren nach Anspruch 1,
**dadurch gekennzeichnet,**
**daß** die gegebenenfalls verbliebene Restfeuchtigkeit (18) durch die Infrarotstrahlung unter Blasenbildung gesammelt wird.

3. Verfahren nach Anspruch 1 oder 2,
**dadurch gekennzeichnet,**
**daß** eine zwischen der Oberfläche und der Kunststoffolie (2) angeordnete Klebstoffschicht (15) durch die Bestrahlung erwärmt wird und gleichmäßig verläuft.

4. Verfahren nach einem der Ansprüche 1 bis 3,
**dadurch gekennzeichnet,**
**daß** vorhandene Ungleichmäßigkeiten der Kunststoffolie (2), wie ungleichmäßige Schichtdicke oder ungleichmäßige Materialstruktur, durch die Bestrahlung zumindest annähernd ausgeglichen werden.

5. Verfahren nach einem der Ansprüche 1 bis 4,
**dadurch gekennzeichnet,**
**daß** die Infrarotstrahlung (12) zumindest Strahlungsanteile, insbesondere ein spektrales Strahlungsflußdichtemaximum, im nahen Infrarot hat.

6. Verfahren zum Beschichten einer PKW-Stoßstange (1) nach einem der Ansprüche 1 bis 5,
**dadurch gekennzeichnet,**
**daß** die Beschichtung (2, 15, 16) insgesamt weniger als 20 Sekunden lang, insbesondere weniger als 10 Sekunden lang bestrahlt wird.

7. Vorrichtung zum Beschichten einer Oberfläche mit einer Kunststoffolie (2), insbesondere der Oberfläche einer PKW-Stoßstange (1), aufweisend:
- eine Strahlungsquelle (11) zum Erzeugen von Infrarotstrahlung (12), um zumindest Teile einer Beschichtung (2, 15, 16) der Oberfläche und/oder beim Beschichten verwendeter Hilfsstoffe (16) zu erwärmen, und
- einen Reflektor (8) zum Erhöhen der Strahlungsflußdichte der von der Strahlungsquelle (11) auf die Beschichtung (2, 15, 16) eingestrahlten Infrarotstrahlung (12)
**dadurch gekennzeichnet, daß**
die Strahlungsquelle (11) zumindest eine Halogenlampe aufweist, die insbesondere bei Oberflächentemperaturen von mehr als 2500 K betreibbar ist.

8. Vorrichtung nach Anspruch 7, weiterhin aufweisend:
- eine Halteeinrichtung (4) zum Halten eines Gegenstandes, insbesondere der PKW-Stoßstange (1), dessen Oberfläche zu beschichten ist, und
- eine Verschiebeeinrichtung (5, 6, 7) zum Verschieben eines auf einen Teilbereich der Beschichtung (2, 15, 16) gerichteten Strahlungsbündels (12) aus der Strahlungsquelle entlang der Oberfläche, um auch andere Teilbereiche der Beschichtung (2, 15, 16) zu bestrahlen.

9. Vorrichtung nach Anspruch 8,
wobei die Verschiebeeinrichtung (5, 6, 7) ein Führungselement (5), insbesondere eine Schiene, aufweist, entlang dem die Strahlungsquelle (11) verschiebbar ist.

10. Vorrichtung nach Anspruch 9,
wobei das Führungselement (5) derart entsprechend der Formgebung des Gegenstandes gebogen ist, daß die Strahlungsquelle (11) bei gleichbleibendem Abstand zu der beschichteten Oberfläche des Gegenstandes verschiebbar ist.

11. Vorrichtung nach einem der Ansprüche 7 bis 10,
wobei die Vorrichtung eine Regeleinrichtung.zum Regeln der Beschichtungstemperatur aufweist und wobei ein auf die Beschichtung (2, 15, 16) ausrichtbares Pyrometer zur Messung der Beschichtungstemperatur vorgesehen ist.

12. Verwendung einer Infrarotlampe (11) zur Qualitätssicherung bei der Ausführung eines Verfahrens zum Beschichten einer Oberfläche mit einer Kunststoffolie (2), insbesondere nach einem der Ansprüche 1 bis 6, wobei die Beschichtung (2, 15, 16) mit Infrarotstrahlung (12) aus der Infrarotlampe (11) bestrahlt wird, um Unregelmäßigkeiten der Beschichtung (2, 15, 16) sichtbar zu machen und/oder zumindest annähernd zu beseitigen.

13. Verwendung nach Anspruch 12,
wobei die Infrarotlampe (11) eine Halogenlampe ist.

14. Verwendung nach Anspruch 12 oder 13,
wobei die Infrarotlampe (11) als Röhrenstrahler mit einem sich linienartig in einer strahlungsdurchlässigen Röhre (9), insbesondere in einer Quarzglasröhre, erstreckenden Glühfaden (10) ausgebildet ist.

15. Verwendung nach Anspruch 14,
wobei die Infrarotlampe (11) mit einem Reflektor (8) kombiniert ist, der sich längs der Röhre (9) erstreckt und diese im Querschnitt derart rinnenartig an der Rückseite umgibt, daß die in Richtung der Vorderseite abgestrahlte Infrarotstrahlung durch reflektierte Strahlung verstärkt wird.

## Claims

1. Method for coating a surface with a plastics sheet (2), in particular the surface of a passenger car bumper (1), wherein the surface and/or the plastics sheet (2) is/are moistened, so that, after applying the plastics sheet (2), the surface is initially separated from the plastics sheet (2) by a film of moisture (16), at least in places, and wherein the film of moisture (16) is at least partly removed by scraping,
**characterised in**
**that** the coating (2, 15, 16) is irradiated with infrared radiation (12) during and/or after scraping in order to heat at least parts of the coating (2, 15, 16) and/or any residual moisture (18) remaining between the surface and the plastics sheet (2).

2. Method according to Claim 1,
**characterised in**
**that** the residual moisture (18) which may remain is collected by the infrared radiation while forming bubbles.

3. Method according to Claim 1 or 2,
**characterised in**
**that** an adhesive layer (15) disposed between the surface and the plastics sheet (2) is heated by the irradiation and extends evenly.

4. Method according to any one of Claims 1 to 3,
**characterised in**
**that** any unevenness of the plastics sheet (2), such as uneven layer thickness or uneven material structure, is at least approximately compensated by the irradiation.

5. Method according to any one of Claims 1 to 4,
**characterised in**
**that** the infrared radiation (12) has at least radiation fractions, in particular a spectral radiation flux density maximum, in the near infrared.

6. Method for coating a passenger car bumper (1) according to any one of Claims 1 to 5,
**characterised in**
**that** the coating (2, 15, 16) is on the whole irradiated for less than 20 seconds, in particular less than 10 seconds.

7. Apparatus for coating a surface with a plastics sheet (2), in particular the surface of a passenger car bumper (1), comprising:
- a radiation source (11) for producing infrared radiation (12) in order to heat at least parts of a coating (2, 15, 16) of the surface and/or auxiliary substances (16) used during coating, and
- a reflector (8) for increasing the radiation flux density of the infrared radiation (12) which is irradiated by the radiation source (11) onto the coating (2, 15, 16),
**characterised in**
**that** the radiation source (11) comprises at least one halogen lamp, which can be operated in particular at surface temperatures exceeding 2500 K.

8. Apparatus according to Claim 7, also comprising:
- a holding device (4) for holding an object, in particular the passenger car bumper (1) whose surface is to be coated, and
- a displacement device (5, 6, 7) for displacing a beam (12), which is directed at a sub-region of the coating (2, 15, 16), from the radiation source along the surface, in order also to irradiate other sub-regions of the coating (2, 15, 16).

9. Apparatus according to Claim 8,
wherein the displacement device (5, 6, 7) comprises a guide element (5), in particular a rail, along which the radiation source (11) can be displaced.

10. Apparatus according to Claim 9,
wherein the guide element (5) is bent according to the shaping of the object such that the radiation source (11) can be displaced at a constant spacing from the surface of the object which is to be coated.

11. Apparatus according to any one of Claims 7 to 10,
wherein the apparatus comprises a regulating device for regulating the coating temperature, and wherein a pyrometer, which can be aligned with the coating (2, 15, 16), is provided to measure the coating temperature.

12. Use of an infrared lamp (11) for quality assurance when carrying out a method for coating a surface with a plastics sheet (2), in particular according to any one of Claims 1 to 6, wherein the coating (2, 15, 16) is irradiated with infrared radiation (12) from the infrared lamp (11) in order to render unevenness of the coating (2, 15, 16) visible and/or at least approximately eliminate such unevenness.

13. Use according to Claim 12,
wherein the infrared lamp (11) is a halogen lamp.

14. Use according to Claim 12 or 13,
wherein the infrared lamp (11) is formed as a tubular radiator with a filament (10), which extends in linear fashion in a radiation-transparent tube (9), in particular in a quartz glass tube.

15. Use according to Claim 14,
wherein the infrared lamp (11) is combined with a reflector (8), which extends along the tube (9) and surrounds the latter at the back like a channel in cross section such that the infrared radiation which is emitted in the direction of the front is reinforced by reflected radiation.

## Revendications

1. Procédé pour recouvrir une surface d'une feuille plastique (2), en particulier la surface d'un pare-chocs (1) d'une voiture de tourisme, dans lequel la surface et/ou la feuille plastique (2) sont humidifiées de manière qu'après application de la feuille plastique (2), la surface soit d'abord au moins séparée de la feuille plastique (2), par endroits, par un film d'humidité (16) et dans lequel le film d'humidité (16) est enlevé au moins partiellement par raclage,
**caractérisé**
**en ce que** le revêtement (2, 15, 16) est exposé à un rayonnement infrarouge (12), pendant et/ou après le raclage, pour chauffer au moins des parties du revêtement (2, 15, 16) et/ou une humidité résiduelle (18) qui subsiste éventuellement entre la surface et la feuille plastique (2).

2. Procédé selon la revendication 1,
**caractérisé**
**en ce que** l'humidité résiduelle (18) qui subsiste éventuellement est recueillie par le rayonnement infrarouge avec formation de bulles.

3. Procédé selon la revendication 1 ou 2,
**caractérisé**
**en ce qu'**une colle de couche (15), disposée entre la surface et la feuille plastique (2), est chauffée par le rayonnement et s'étend uniformément.

4. Procédé selon l'une des revendications 1 à 3,
**caractérisé**
**en ce que** des irrégularités existantes de la feuille plastique (2), telles qu'une épaisseur de couche irrégulière ou une structure irrégulière de la matière, sont approximativement compensées par le rayonnement.

5. Procédé selon l'une des revendications 1 à 4,
**caractérisé**
**en ce que** le rayonnement infrarouge (12) possède au moins des composantes de rayonnement, en particulier un maximum spectral de densité de flux de rayonnement, dans l'infrarouge proche.

6. Procédé pour recouvrir d'un revêtement un pare-chocs (1) d'une voiture de tourisme selon l'une des revendications 1 à 5,
**caractérisé**
**en ce que** dans l'ensemble le revêtement (2, 15, 16) est exposé à un rayonnement pendant moins de 20 secondes, en particulier moins de 10 secondes.

7. Dispositif pour recouvrir une surface d'une feuille plastique (2), en particulier la surface d'un pare-chocs (1) d'une voiture de tourisme, comportant :
- une source de rayonnement (11) destinée à produire un rayonnement infrarouge (12), pour chauffer au moins des parties d'un revêtement (2, 15, 16) de la surface et/ou des adjuvants (16) utilisés pendant l'application du revêtement, et
- un réflecteur (8) pour accroître la densité de flux de rayonnement du rayonnement infrarouge émis par la source de rayonnement (11) sur le revêtement (2, 15, 16)
**caractérisé en ce que**
la source de rayonnement (11) comporte au moins une lampe halogène qui peut fonctionner en particulier à des températures superficielles supérieures à 2 500 K.

8. Dispositif selon la revendication 7, comportant en outre :
- un dispositif de maintien (4) pour maintenir un objet, en particulier le pare-chocs (1) de la voiture de tourisme dont la surface doit être recouverte, et
- un dispositif de déplacement (5, 6, 7) pour déplacer un faisceau de rayons (12), dirigé sur une zone partielle du revêtement (2, 15, 16) provenant de la source de rayonnement, le long de la surface, afin d'exposer au rayonnement aussi d'autres zones partielles du revêtement (2, 15, 16).

9. Dispositif selon la revendication 8,
dans lequel le dispositif de déplacement (5, 6, 7) comporte un élément de guidage (5), en particulier un rail, le long duquel la source de rayonnement (11) peut être déplacée.

10. Dispositif selon la revendication 9,
dans lequel l'élément de guidage (5) est cintré conformément à la forme de l'objet, de manière que la source de rayonnement (11) puisse être déplacée à distance constante par rapport à la surface recouverte de l'objet.

11. Dispositif selon l'une des revendications 7 à 10,
dans lequel le dispositif comporte un dispositif de régulation pour la régulation de la température de revêtement et dans lequel il est prévu un pyromètre orientable vers le revêtement (2, 15, 16), pour la mesure de la température de revêtement.

12. Utilisation d'une lampe infrarouge (11) pour l'assurance qualité dans la mise en oeuvre d'un procédé destiné à recouvrir une surface d'une feuille plastique (2), en particulier selon l'une des revendications 1 à 6, dans laquelle le revêtement (2, 15, 16) est exposé à un rayonnement infrarouge (12) provenant de la lampe infrarouge (11), afin de rendre visibles des irrégularités du revêtement (2, 15, 16) et/ou les éliminer au moins approximativement.

13. Utilisation selon la revendication 12,
dans laquelle la lampe infrarouge (11) est une lampe halogène.

14. Utilisation selon la revendication 12 ou 13,
dans laquelle la lampe infrarouge (11) est conformée en tube radiant avec un fil incandescent (10) s'étendant en ligne dans un tube (9) perméable au rayonnement, en particulier dans un tube en verre au quartz.

15. Utilisation selon la revendication 14,
dans laquelle la lampe infrarouge (11) est combinée à un réflecteur (8) qui s'étend le long du tube (9) et entoure celui-ci dans la section transversale, à la manière d'une rigole, sur la face arrière, de manière que le rayonnement infrarouge, émis en direction de la face avant, soit intensifié par un rayonnement réfléchi.
